# EUROPEAN PATENT SPECIFICATION

(11) **EP 4 001 881 B1**
(45) Date of publication and mention of the grant of the patent: **09.07.2025**
(21) Application number: 21208574.0
(22) Date of filing: 16.11.2021
(51) Int. Cl.: G01N 1/22, B01L 7/00, B64D 11/04, B64D 11/00

(54) **SYSTEMS AND METHODS OF OBTAINING A BIOLOGICAL SAMPLE REPRESENTATIVE OF A PASSENGER CABIN ON AN AIRCRAFT USING AN AIR CYCLONIC COLLECTOR**
SYSTEME UND VERFAHREN ZUR ENTNAHME EINER FÜR DIE PASSAGIERKABINE REPRÄSENTATIVEN BIOLOGISCHEN PROBE IN EINEM FLUGZEUG UNTER VERWENDUNG EINES LUFTZYKLONSAMMLERS
SYSTÈMES ET PROCÉDÉS D'OBTENTION D'UN ÉCHANTILLON BIOLOGIQUE REPRÉSENTATIF DE LA CABINE DE PASSAGERS D'UN AÉRONEF À L'AIDE D'UN COLLECTEUR D'AIR CYCLONIQUE

(30) Priority: 16.11.2020 US 202063114350 P; 16.11.2020 US 202063114330 P; 16.11.2020 US 202063114339 P; 16.11.2020 US 202063114400 P; 16.11.2020 US 202063114064 P; 16.11.2020 US 202063114157 P; 16.11.2020 US 202063114386 P; 16.11.2020 US 202063114366 P
(43) Date of publication of application: 25.05.2022
(73) Proprietor: B/E Aerospace, Inc., Winston-Salem, NC 27105 (US)
(72) Inventor: GONZALEZ, Arnau Castillo, 3607 AK Maarseen (NL); MURCIA, Antonio Martinez, CP 03317 Orihuela (ES); SURAWSKI, Eric, Hebron, CT 06248 (US)
(74) Representative: Dehns

(56) References cited:
- US-A1- 2004 038 385
- US-A1- 2008 245 706
- US-A1- 2011 159 596
- US-A1- 2012 329 142
- US-A1- 2013 040 406
- US-A1- 2013 137 183
- US-A1- 2016 025 603
- US-A1- 2016 193 603
- ANONYMOUS: "Compendium of Methods for the Determination of Toxic Organic Compounds in Ambient Air Second Edition Compendium Method TO-4A Determination of Pesticides and Polychlorinated Biphenyls in Ambient Air Using High Volume Polyurethane Foam (PUF) Sampling Followed by Gas Chromatographic/Multi-Detector Dete", 1 January 1999 (1999-01-01), pages 1 - 53, XP055906647, Retrieved from the Internet <URL:https://www.epa.gov/sites/default/files/2016-02/documents/to-4ar2r.pdf> [retrieved on 20220329]

## Description

### Background

### Technological Field

The present application is related to a system and method used to collect a representative air sample of an aircraft, more specifically to a method and systems for collecting a biological sample on an aircraft using a collector.

### Description of Related Art

The spread progression of SARS-CoV-2 around the world has risen a red flag: economic globalization creates systemic risks. The CoVID-19 pandemic shed light on the need for better monitoring, detecting, and isolating ill passengers, specifically due to the detrimental impact on the global economy, specifically air travel due to closed borders, movement restrictions, and testing requirements.

However, the CoVID-19 pandemic the air travel industry has proven that air travel can be safe and that aircraft cabins have a well-managed airflow that minimize the risk for transmission of virus, and that being seated onboard an aircraft is safer than shopping in large stores. Governments and other authorities need to assume that aircraft are contaminated until proven "clean", as 25% of COVID-19 cases are asymptomatic or pre-symptomatic; but still contagious. Thus, if borders shutdown and a drastic reduction in international travel global passenger travel is greatly reduced. To date travelers and governments have relied on individual diagnostic tests. The uncertainty of the results has reduced people's inclination to travel and subsequent airline inclination to maintain routes. However, to date, the microbial control of environment (air) has received very little attention, if any.

Accordingly, there is still a need in the art for developing and implementing highly precise systems and methods for pathogen detection, adapted to the aerospace segment (airports, aircraft, etc.). The present disclosure provides a solution for this need. US 2004/038385 A1 describes a system for autonomous monitoring of bioagents. US 2011/159596 A1 describes a substance detector.

### Summary of the Invention

A system for sampling and monitoring aircraft air is disclosed and defined in claim 1. The system contains buffer and a reagent fluid which includes molecular grade sterile water and a range of components as salts, surfactants, and different additives for nucleic acid protection and conservation. Buffer composition and pH range are determined depending of quality and quantity of samples to be harvested.

The inlet of the conical main body can have a smaller diameter than the outlet. The conical main body can taper down away from the inlet. The HEPA filter can be positioned in the recirculation flow path downstream of the collector, wherein the HEPA filter is configured and adapted to clean air flowing through the recirculation flow path after the air passes through the collector. The particles can include aerosol droplets exhaled from passengers throughout at least a portion of a flight. The system can include mounting slot in the outlet flow path upstream from the outflow valve, wherein the collector is positioned within the mounting slot. The collector can be configured and adapted for the conical sampling-recipient to be removed from the mounting slot for testing.

The vessel can include a plurality of vessels affixed within a space between an aircraft hull and a passenger cabin floor for collecting air samples at a plurality of locations throughout the aircraft. The plurality of vessels can be affixed between passenger cabin seats, within at least one of: galley areas, lavatories, or corridors, or be attached to a galley trolley. At least one of the plurality of vessels can be located within an aft cargo bay area.

A method of monitoring aircraft air using the system of claim 1 is also disclosed and defined by claim 10. The method includes driving ambient cabin air from the cabin through a reagent fluid located within a conical vessel of the system of claim 1, concentrating the reagent fluid, extracting and purifying the genetic material (DNA/RNA) from a portion of the concentrated reagent fluid to provide a sample for a test. The system can include conducting a Polymerase Chain Reaction (PCR) test on the testable sample, including end-point PCR (epPCR), or/and real time quantitative PCR (qPCR), or/and Reverse transcriptase-quantitative PCR (RT-qPCR). The ambient cabin air can pass through a HEPA-filter after passing through the reagent fluid.

Extracting and purifying includes can include passing the concentrated reagent fluid through silica columns. Extracting and purifying can include passing the concentrated reagent fluid through magnetized beads. Also by passing the sample through a micro-fluidic multi-channel subjected to several chemical-protocol steps, such as concentrating the reagent fluid can include a passing the reagent fluid through a concentration pipette, also a concentrator using specific foams.

These and other features of the systems and methods of the subject disclosure will become more readily apparent to those skilled in the art from the following detailed description of the preferred embodiments taken in conjunction with the drawings.

### Brief Description of the Drawings

So that those skilled in the art to which the subject disclosure appertains will readily understand how to make and use the devices and methods of the subject disclosure without undue experimentation, embodiments thereof will be described in detail herein below with reference to certain figures, wherein:
FIG. 1 is schematic view of the conical collector; and
FIG. 2 is a schematic view of an aircraft showing diagrammatically where the conical collector of FIG. 1 are be located.

### Detailed Description

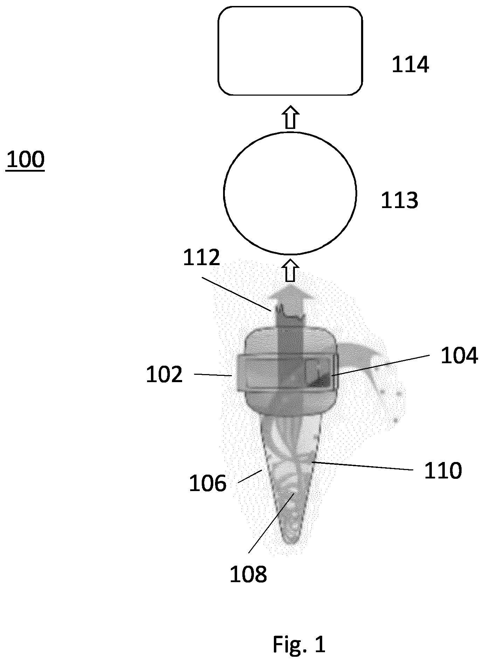

Reference will now be made to the drawings wherein like reference numerals identify similar structural features or aspects of the subject disclosure. For purposes of explanation and illustration, and not limitation, a partial view of an exemplary embodiment of a system in accordance with the disclosure is shown in FIG. 1 and is designated generally by reference character 100. The global collector system described below is used to collect a bulk sample, representative of all passengers as a group on the aircraft and to test it to provide a bulk screening of the aircraft.

FIG. 1 shows a system 100 for monitoring aircraft air. The system 100 includes a vessel 102 having an inlet 104, a conical main body 106 containing: a reagent fluid 108 for extracting particles from the air coating an inner surface 110 on the conical main body, and an outlet 112. The outlet 112 is positioned within either an outlet flow path or a recirculation flow path of the aircraft. An outflow valve 114 (shown diagrammatically) is positioned in the outlet flow path downstream from the collector or a HEPA filter 113 (also shown diagrammatically) can be positioned in the recirculation flow path downstream from the collector. This reagent fluid 108 is customized for the targeted pathogen/contaminant as well to the diagnostic test. Referring further to FIG. 1, the inlet 104 of the conical main body 102 has a smaller diameter (d1) than the diameter of the outlet 112 (d2). The conical main body 102 tapers down away from the inlet 104.

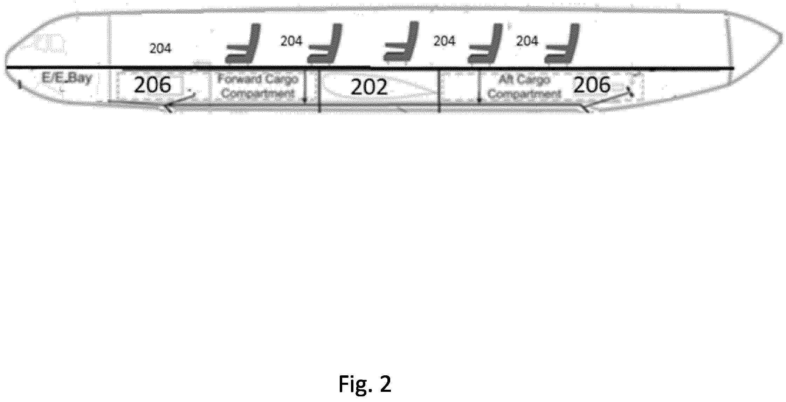

Referring to FIG. 2, a plurality of vessels 102 can be affixed within a space 202 between an aircraft hull and a passenger cabin floor for collecting air samples. The vessels can be affixed between passenger cabin seats 204, within at least one of: galley areas, lavatories, or corridors, or be attached to a galley trolley. At least one of the plurality of vessels can be located within an aft or fwd cargo bay area 206.

A method of monitoring aircraft air is also disclosed. The method includes driving ambient cabin air from the cabin through a reagent fluid located within the conical vessel, concentrating the reagent fluid, extracting and purifying a portion of the concentrated reagent fluid to produce a testable sample to prepare the sample for a test, and conducting a pathogen/contaminant diagnostic test such as a Polymerase Chain Reaction (PCR) test on the testable sample.

The methods and systems of the present disclosure, as described above and shown in the drawings, provide for an improved bulk data and analysis of passenger pathogens on an aircraft. While the apparatus and methods of the subject disclosure have been shown and described with reference to preferred embodiments, those skilled in the art will readily appreciate that changes and/or modifications may be made thereto without departing from the scope of the claims.

## Claims

1. A system (100) for monitoring aircraft air via a diagnostic test comprising:
a vessel (102) having an inlet (104), a conical main body (106) containing: a reagent fluid (108) for extracting particles from the air coating an inner surface (110) on the conical main body, and an outlet (112), wherein the outlet is positioned within at least one of an outlet flow path or a recirculation flow path of an aircraft; and
wherein the system further comprises at least one of an outflow valve (114) positioned in the outlet flow path downstream from the vessel or a HEPA filter (113) positioned in the recirculation flow path downstream from the vessel and wherein the reagent fluid (108) includes a customized mixture for the targeted pathogen or contaminant and diagnostic test;
the system further containing a buffer;
and wherein the reagent fluid includes molecular grade sterile water and a range of components for nucleic acid protection and conservation, and
the buffer composition and pH range are varied depending on quality and quantity of samples to be harvested.

2. The system of claim 1, wherein the inlet (104) has a smaller diameter than the outlet (112).

3. The system of any preceding claim, wherein the conical main body (106) tapers down away from the inlet (104).

4. The system of any preceding claim, wherein the HEPA filter (113) is positioned in the recirculation flow path downstream of the vessel, wherein the HEPA filter is configured and adapted to filter the air flowing through the recirculation flow path.

5. The system of any preceding claim, wherein the particles include aerosol droplets exhaled from passengers throughout at least a portion of a flight.

6. The system of any preceding claim, further comprising a mounting slot in the outlet flow path upstream from the outflow valve, wherein the vessel is positioned within the mounting slot; and optionally wherein the vessel is configured and adapted to be removed from the mounting slot for testing.

7. The system of any preceding claim, wherein the vessel includes a plurality of vessels (102) affixed within a space (202) between an aircraft hull and a passenger cabin floor for collecting air samples.

8. The system of claim 7, wherein the plurality of vessels are affixed between passenger cabin seats (204).

9. The system of claim 8, wherein at least one of the plurality of vessels is affixed within at least one of: galley areas, lavatories, or corridors; and/or wherein at least one of the plurality of vessels is attached to a galley trolley; and/or wherein at least one of the plurality of vessels is located within an cargo bay area.

10. A method of monitoring aircraft air using the system of claim 1, the method comprising:
driving ambient cabin air from the cabin through a reagent fluid (108) located within the conical vessel of the system of claim 1;
concentrating the reagent fluid; and
extracting and purifying a portion of the concentrated reagent fluid to produce a testable sample; and further comprising conducting a pathogen/contaminant diagnostic test, on the testable sample and wherein the reagent fluid (108) includes a customized mixture for the targeted pathogen or contaminant and diagnostic test.

11. The method of claim 10, wherein the ambient cabin air passes through a HEPA-filter after passing through the reagent fluid.

12. The method of claim 10 or 11, wherein extracting and purifying includes passing the concentrated reagent fluid through silica columns; and/or wherein extracting and purifying includes passing the concentrated reagent fluid through magnetic beads; and/or wherein extracting and purifying includes passing the concentrated reagent fluid through a microfluidic system.

13. The method of any of claims 10 to 12, wherein concentrating the reagent fluid includes a passing the reagent fluid through a concentrator with foam.

## Patentansprüche

1. System (100) zum Überwachen der Luft in einem Flugzeug über einen Diagnosetest, umfassend:
einen Behälter (102), der einen Einlauf (104), einen konischen Hauptkörper (106), enthaltend: ein Reagenzfluid (108) zum Extrahieren von Partikeln aus der eine Innenfläche (110) des konischen Hauptkörpers bedeckenden Luft, und einen Ablauf (112) aufweist, wobei der Ablauf innerhalb mindestens eines von einem Ablaufströmungsweg oder einem Umwälzströmungsweg eines Flugzeugs positioniert ist; und
wobei das System ferner mindestens eines von einem in dem Ablaufströmungsweg stromabwärts von dem Behälter positionierten Auslassventil (114) oder einem in dem Umwälzströmungsweg stromabwärts von dem Behälter positionierten HEPA-Filter (113) umfasst und wobei das Reagenzfluid (108) eine aufgabenspezifische Mischung für den Zielerreger oder den Zielschadstoff und den Diagnosetest beinhaltet;
wobei das System ferner einen Puffer enthält;
und wobei das Reagenzfluid steriles Wasser in molekularbiologischer Qualität und eine Reihe von Komponenten zum Schutz und zur Konservierung von Nukleinsäure beinhaltet und die Pufferzusammensetzung und der pH-Bereich abhängig von Qualität und Quantität der zu entnehmenden Proben variieren.

2. System nach Anspruch 1, wobei der Einlauf (104) einen kleineren Durchmesser aufweist als der Ablauf (112).

3. System nach einem der vorhergehenden Ansprüche, wobei sich der konische Hauptkörper (106) von dem Einlauf (104) weg nach unten verjüngt.

4. System nach einem der vorhergehenden Ansprüche, wobei das HEPA-Filter (113) in dem Umwälzströmungsweg stromabwärts des Behälters positioniert ist, wobei das HEPA-Filter zum Filtern der durch den Umwälzströmungsweg strömenden Luft konfiguriert und angepasst ist.

5. System nach einem der vorhergehenden Ansprüche, wobei die Partikel Aerosoltröpfchen beinhalten, die von Passagieren während mindestens eines Teils eines Fluges ausgeatmet werden.

6. System nach einem der vorhergehenden Ansprüche, ferner umfassend einen Montageschlitz in dem Ablaufströmungsweg stromaufwärts von dem Auslassventil, wobei der Behälter innerhalb des Montageschlitzes positioniert ist; und wobei der Behälter optional zum Entfernen aus dem Montageschlitz zum Testen konfiguriert und angepasst ist.

7. System nach einem der vorhergehenden Ansprüche, wobei der Behälter eine Vielzahl von Behältern (102) zum Sammeln von Luftproben beinhaltet, die innerhalb eines Raums (202) zwischen einem Flugzeugrumpf und einem Boden der Passagierkabine befestigt ist.

8. System nach Anspruch 7, wobei die Vielzahl von Behältern zwischen Sitzen der Passagierkabine (204) befestigt ist.

9. System nach Anspruch 8, wobei mindestens einer der Vielzahl von Behältern innerhalb mindestens eines der Folgenden befestigt ist: Bordküchenbereiche, Toiletten oder Korridore; und/oder wobei mindestens einer der Vielzahl von Behältern an einem Bordküchenwagen befestigt ist; und/oder wobei sich mindestens einer der Vielzahl von Behältern innerhalb eines Frachtraumbereich befindet.

10. Verfahren zum Überwachen der Luft in einem Flugzeug unter Verwendung des Systems nach Anspruch 1, wobei das Verfahren Folgendes umfasst:
Leiten von Umgebungskabinenluft durch ein Reagenzfluid (108), das sich innerhalb des konischen Behälters des Systems nach Anspruch 1 befindet;
Konzentrieren des Reagenzfluids; und
Extrahieren und Reinigen eines Teils des konzentrierten Reagenzfluids, um eine testbare Probe zu produzieren; und ferner umfassend Durchführen eines Diagnosetests auf Erreger/Schadstoff mit der testbaren Probe und wobei das Reagenzfluid (108) eine aufgabenspezifische Mischung für den Zielerreger oder den Zielschadstoff und den Diagnosetest beinhaltet.

11. Verfahren nach Anspruch 10, wobei die Umgebungskabinenluft nach Passieren des Reagenzfluids durch ein HEPA-Filter passiert.

12. Verfahren nach Anspruch 10 oder 11, wobei das Extrahieren und Reinigen Passieren des konzentrierten Reagenzfluids durch Kieselsäuresäulen beinhaltet; und/oder wobei das Extrahieren und Reinigen Passieren des konzentrierten Reagenzfluids durch Magnetkügelchen beinhaltet; und/oder wobei das Extrahieren und Reinigen Passieren des konzentrierten Reagenzfluids durch ein Mikrofluidsystem beinhaltet.

13. Verfahren nach einem der Ansprüche 10 bis 12, wobei das Konzentrieren des Reagenzfluids Passieren des Reagenzfluids durch einen Konzentrator mit Schaum beinhaltet.

## Revendications

1. Système (100) de surveillance de l'air d'un aéronef par l'intermédiaire d'un test de diagnostic comprenant :
un récipient (102) présentant une entrée (104), un corps principal conique (106) contenant : un fluide réactif (108) destiné à extraire les particules de l'air recouvrant une surface intérieure (110) du corps principal conique, et une sortie (112), dans lequel la sortie est positionnée à l'intérieur d'au moins l'un d'un trajet d'écoulement de sortie ou d'un trajet d'écoulement de recirculation d'un aéronef ; et
dans lequel le système comprend également au moins l'un ou l'une d'une vanne de sortie (114) positionnée dans le trajet d'écoulement de sortie en aval du récipient ou d'un filtre HEPA (113) positionné dans le trajet d'écoulement de recirculation en aval du récipient et dans lequel le fluide réactif (108) comporte un mélange personnalisé pour le pathogène ou le contaminant ciblé et le test de diagnostic ;
le système contenant en outre un tampon ;
et dans lequel le fluide réactif comporte de l'eau stérile de qualité moléculaire et une plage de composants pour la protection et la conservation des acides nucléiques, et
la composition du tampon et la plage de pH varient en fonction de la qualité et de la quantité des échantillons à récolter.

2. Système selon la revendication 1, dans lequel l'entrée (104) présente un diamètre plus petit que la sortie (112).

3. Système selon une quelconque revendication précédente, dans lequel le corps principal conique (106) se rétrécit vers le bas en s'éloignant de l'entrée (104).

4. Système selon une quelconque revendication précédente, dans lequel le filtre HEPA (113) est positionné dans le trajet d'écoulement de recirculation en aval du récipient, dans lequel le filtre HEPA est configuré et adapté pour filtrer l'air circulant à travers le trajet d'écoulement de recirculation.

5. Système selon une quelconque revendication précédente, dans lequel les particules comportent des gouttelettes d'aérosol expirées par les passagers pendant au moins une partie d'un vol.

6. Système selon une quelconque revendication précédente, comprenant également une fente de montage dans le trajet d'écoulement de sortie en amont de la vanne de sortie, dans lequel le récipient est positionné à l'intérieur de la fente de montage ; et éventuellement dans lequel le récipient est configuré et adapté pour être retiré de la fente de montage pour être testé.

7. Système selon une quelconque revendication précédente, dans lequel le récipient comporte une pluralité de récipients (102) fixés à l'intérieur d'un espace (202) entre une coque d'aéronef et un plancher de cabine de passagers pour collecter des échantillons d'air.

8. Système selon la revendication 7, dans lequel la pluralité de récipients sont fixés entre les sièges de cabine de passagers (204).

9. Système selon la revendication 8, dans lequel au moins l'un de la pluralité de récipients est fixé à l'intérieur d'au moins l'une des zones suivantes : zones d'office, toilettes ou couloirs ; et/ou dans lequel au moins l'un de la pluralité de récipients est fixé à un chariot d'office ; et/ou dans lequel au moins l'un de la pluralité de récipients est situé à l'intérieur d'une zone de soute.

10. Procédé de surveillance de l'air d'un aéronef utilisant le système selon la revendication 1, le procédé comprenant :
l'entraînement de l'air ambiant de la cabine depuis la cabine à travers un fluide réactif (108) situé à l'intérieur du récipient conique du système selon la revendication 1 ;
la concentration du fluide réactif ; et
l'extraction et la purification d'une partie du fluide réactif concentré pour produire un échantillon testable ; et comprenant également la réalisation d'un test de diagnostic d'agent pathogène/de contaminant sur l'échantillon testable et dans lequel le fluide réactif (108) comporte un mélange personnalisé pour l'agent pathogène ou le contaminant ciblé et le test de diagnostic.

11. Procédé selon la revendication 10, dans lequel l'air ambiant de la cabine traverse un filtre HEPA après avoir traversé le fluide réactif.

12. Procédé selon la revendication 10 ou 11, dans lequel l'extraction et la purification comportent le passage du fluide réactif concentré à travers des colonnes de silice ; et/ou dans lequel l'extraction et la purification comportent le passage du fluide réactif concentré à travers des billes magnétiques ; et/ou dans lequel l'extraction et la purification comportent le passage du fluide réactif concentré à travers un système microfluidique.

13. Procédé selon l'une quelconque des revendications 10 à 12, dans lequel la concentration du fluide réactif comporte un passage du fluide réactif à travers un concentrateur avec de la mousse.
